# EUROPÄISCHE PATENTANMELDUNG

(11) **EP 2 676 947 A1**
(43) Veröffentlichungstag der Anmeldung: **25.12.2013**
(21) Anmeldenummer: 12172384.5
(22) Anmeldetag: 18.06.2012
(51) Int. Cl.: C07C 17/358, C07C 25/02, C07C 25/08, C07C 25/10

(54) **Verfahren zur Isomerisierung von subsituierten Aromaten unter Verwendung einer Salzschmelze**

(71) Anmelder: LANXESS Deutschland GmbH, 50569 Köln (DE)
(72) Erfinder: Kuhlmann, Sven, Dr., 50733 Köln (DE); Weber, Hans-Martin, Dr., 51373 Leverkusen (DE); Böger, Uwe, 51375 Leverkusen (DE); Wasserscheid, Peter, Prof. Dr., 91054 Erlangen (DE); Schlenk, Stefan, 91054 Erlangen (DE); Messner, Julia, 90478 Erlangen (DE)

(57) **Zusammenfassung**

Die Erfindung betrifft ein Verfahren zur Isomerisierung von substituierten aromatischen Verbindungen in Gegenwart spezieller Salzschmelzen. Beispielhaft seien genannt die Isomerisierung von Dichlorbenzol, Trichlorbenzol, Chlortoluol oder Chlorbrombenzol unter Verwendung von Salzschmelzen bestehend aus AlCl3/LiCl, AlCl3/LiBr oder AlBr3/LiBr.

## Beschreibung

Die Erfindung betrifft ein Verfahren zur Isomerisierung von substituierten aromatischen Verbindungen in Gegenwart spezieller Salzschmelzen.

Die Isomerisierung ist eine in der Technik verbreitet angewandte Methode zur Änderung des Substitutionsmusters von Verbindungen durch zum Beispiel katalytische Überführung eines Isomeren oder Isomerengemisches in ein anderes. Dabei stellt sich üblicherweise ein der Reaktionstemperatur entsprechendes, thermodynamisches Gleichgewicht ein, welches von der Zusammensetzung des urspünglich eingesetzten Isomeren oder Isomerengemisches abweichen kann.

Mehrfach substituierte, aromatische Verbindungen besitzen eine hohe industrielle Bedeutung. Allerdings werden über ihre typischen Herstellungsverfahren meist Isomerengemische erhalten, deren zusammensetzung nicht immer den Markterfordernissen entsprechen. So werden beispielsweise bei der Dichlorierung von Benzol typischerweise nur 1 bis 3 Gew.-% meta-Dichlorbenzol gewonnen, neben typischerweise 55 bis 70 Gew.-% para-Dichlorbenzol und typischerweise 28 bis 43 Gew.-% ortho-Dichlorbenzol. Eine Isolierung der geringen Mengen meta-Dichlorbenzols aus dem Chlorierungsgemisch ist sehr aufwendig.

Es ist bekannt, das aromatische Verbindungen mit Halogensubstituenten mit Hilfe von sauren Katalysatoren zum thermodynamischen Gleichgewicht isomerisiert werden können. Aromatische Verbindungen mit Halogensubstituenten können dabei zum Beispiel Dichlorbenzolgemische sein.

Als Katalysatoren eignen sich unter anderem saure Zeolite (Russ. Chem. Bull. 1994, 43(11), 1809-1811) oder Aluminiumchlorid, welches durch partielle Hydrolyse oder durch Säurezusatz saure Protonen enthält. So beschreibt DE 1020323 A die kontinuierliche Isomerisierung von Dichlorbenzolgemischen enthaltend para- und ortho-Dichlorbenzol mit Hilfe von Aluminiumchlorid in Kombination mit Trägermaterialien und in Gegenwart eines Clorwasserstoff-Gasstromes. Dabei wird entstandenes meta-Dichlorbenzol über eine mit dem Reaktionsgefäß verbundene Rektifikationskolonne abgetrennt. Desaktivierter Katalysator wird fortlaufend abgetrennt und ersetzt.

In J. Org. Chem. 1962, 27, 3449-3455 wird die Verwendung von wasseraktivierten Aluminiumchlorid-Katalysatoren zur Isomerisierung von Dichlorbenzolen beschrieben. Hier wird ein sauer katalysierter intramolekularer 1,2-Chloridshift als Mechanismus der Isomerisierung vorgeschlagen.

DE 4430950 A beschreibt ein verbessertes Isomerisierungsverfahren, bei dem die Aktivität des Aluminiumchlorid-Katalysators durch Zusatz von speziellen wasserspendenden Aktivatoren erhöht wird.

Den Verfahren gemäß Stand der Technik ist jedoch gemein, dass die Abtrennung des Katalysators sowie der Erhalt der katalytischen Aktivität problematisch sind. Dies führt dazu, dass ein nicht unerheblicher Anteil des Katalysators hydrolysiert und ersetzt werden muss. Die hierbei anfallenden Kosten für Abwasser und Katalysator sind wesentliche Nachteile der beschriebenen Verfahren.

Vor dem Hintergrund des vorstehend genannten Standes der Technik bestand daher die Aufgabe, ein besonders wirtschaftliches Verfahren bereitzustellen, das die effiziente Isomerisierung und Produktaufarbeitung erlaubt.

Gegenstand der Erfindung ist nun ein Verfahren zur Isomerisierung von Verbindungen der Formel (I) oder Mischungen von Verbindungen der Formel (I)

Ar-Rₙ (I)

worin
- Ar: für einen n-valenten aromatischen Rest steht
- n: für eine natürliche Zahl von 2 oder mehr, vorzugsweise 2 oder 3 und besonders bevorzugt 2 steht und
- R: jeweils unabhängig für Halogen, Alkyl, Fluoralkyl, Aryl, Alkyl-Aryl oder Amino steht, das dadurch gekennzeichnet ist, dass die Isomerisierung in Gegenwart einer Salzschmelze erfolgt, die enthält:

● zumindest eine Verbindung der Formel (II):

   [M¹][X¹]ₘ₁

   in der
   - M¹: für Aluminium, Gallium, Indium, Kupfer, Eisen, Cobalt, Nickel oder Kupfer,
   - X¹: für ein Halogen, vorzugsweise Chlor oder Brom, besonders bevorzugt Chlor steht und
   - m1: für Aluminium, Gallium, Indium, Eisen für 3, für Cobalt, Nickel und Kupfer für 2 steht
● zumindest eine Verbindung der Formel (III):

   [M²][X²]ₘ₂

   in der
   - M²: für ein Erdalkali- oder Alkalimetall, vorzugsweise ein Alkalimetall und besonders bevorzugt für Lithium, Natrium oder Kalium steht, wobei Lithium noch weiter bevorzugt ist,
   - X²: für ein Halogen, vorzugsweise Chlor oder Brom, besonders bevorzugt Chlor steht und
   - m2: für Erdalkalimetalle für 2, für Alkalimetalle für 1 steht.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

In Formel (I) steht
- Ar: bevorzugt für carbocyclische aromatische Reste mit 6 bis 24 Gerüstkohlenstoffatomen oder für heteroaromatische Reste mit 5 bis 24 Gerüstatomen, in denen keines, ein, zwei oder drei Gerüstatome pro Cyclus, im gesamten Molekül mindestens jedoch ein Gerüstatom Heteroatome sind, die ausgewählt sind aus der Gruppe Stickstoff, Schwefel oder Sauerstoff.
- Ar: steht besonders bevorzugt für aromatische Reste die sich vom Benzol, Naphtalin, Phenanthren, Anthracen, Biphenyl, Binaphthyl, Fluoren, Pyridin, Oxazol, Thiophen, Benzofuran, Benzothiophen, Dibenzofuran, Dibenzothiophen, Furan, Indol, Pyridazin, Pyrazin, Pyrimidin, Triazol oder Chinolin ableiten
- Ar: steht ganz besonders bevorzugt für aromatische Reste, die sich vom Benzol oder Naphthalin ableiten, wobei ein aromatischer Rest der sich von Benzol ableitet, noch weiter bevorzugt ist.

Unter Ableitung ist hierbei zu verstehen, dass es sich um einen n-valenten Rest aus der ansonsten unsubstituierten aromatischen Verbindung handelt. Der trivalente aromatische Rest zum Beispiel im Falle von Trichlorbenzol leitet sich vom Benzol ab.

In Formel (I) steht Alkyl jeweils unabhängig für einen geradkettigen, cyclischen, verzweigten oder unverzweigten Alkyl-Rest.

Beispielsweise steht Alkyl für Methyl, Ethyl, n-Propyl, Isopropyl, n-Butyl, tert.-Butyl, n-Pentyl, Cyclohexyl, n-Hexyl, n-Heptyl, n-Octyl, iso-Octyl, n-Decyl oder n-Dodecyl.

In Formel (I) steht Fluoralkyl jeweils unabhängig für einen geradkettigen, cyclischen, verzweigten oder unverzweigten Alkyl-Rest, der durch mindestens ein Fluoratom substituiert ist.

Beispielsweise steht Fluoralkyl für Fluormethyl, Difluormethyl, Trifluormethyl, 2,2,2-Trifluorethyl, Pentafluorethyl, Heptafluorisopropyl oder Nonafluor-n-butyl.

Bevorzugte Verbindungen sind solche, in denen n = 2 ist und ein oder zwei der Reste Halogene wie vorzugsweise Brom oder Chlor, besonders bevorzugt Chlor sind sowie Verbindungen, in denen n = 3 ist und zwei oder drei der Reste Halogene wie vorzugsweise Brom oder Chlor, besonders bevorzugt Chlor sind.

Besonders bevorzugte Verbindungen sind die jeweiligen Isomere von Dichlorbenzol, vorzugsweise p-Dichlorbenzol und o-Dichlorbenzol, Trichlorbenzol, Chlortoluol, Dibrombenzol und Bromchlorbenzol. Besonders bevorzugt sind die Isomere von Dichlorbenzol.

Unter einer Salzschmelze ist erfindungsgemäß eine Flüssigkeit zu verstehen, die zu 90 Gew.-% oder mehr, bevorzugt zu 95 Gew.-% oder mehr, besonders bevorzugt zu 98 Gew.-% oder mehr aus den Verbindungen der Formel (II) und (III) besteht.

Der von 100 % abweichende Anteil kann sich zum Beispiel aus der nicht vollkommenen Reinheit der eingesetzten Verbindungen ergeben, die beispielsweise technische Qualität besitzen können. Das bringt wirtschaftliche Vorteile mit sich.

Dem Fachmann ist klar, dass sich aus den Verbindungen der Formeln (II) und (III) zum Beispiel Doppelsalze der Formel [M¹][M²]([X¹]ₘ₁[X²]ₘ₂) wie zum Beispiel LiAlCl₄ oder Tripelsalze wie NaAl₂Cl₇ oder noch höhere Komplexsalze bilden können. Solche Verbindungen sind unter dem Begriff Salzschmelze enthaltend zumindest eine Verbindung der Formel (II) und zumindest eine Verbindung der Formel (III) subsumiert bzw solche Verbindungen erfüllen das Merkmal "enthaltend zumindest eine Verbindung der Formel (II) und zumindest eine Verbindung der Formel (III)".

Dem Fachmann ist weiterhin klar, dass je nach Zusammensatzung dieser Salzschmelzen der Schmelzpunkt variieren kann.

Vorzugsweise beträgt daher das molare Verhältnis von Verbindungen der Formel (II) zu Verbindungen der Formel (III) 1:1 1 bis 10:1, vorzugsweise 1:1 1 bis 3:1 und besonders bevorzugt 1,8:1 bis 2,2:1, wobei 2:1 noch weiter bevorzugt ist.

Bevorzugte Verbindungen der Formel (II) sind Aluminiumchlorid (AlCl₃) , Aluminiumbromid (AlCl₃) und Galliumchlorid (GaCl₃), wobei Aluminiumchlorid bevorzugt ist.

Bevorzugte Verbindungen der Formel (III) sind Lithiumchlorid (LiCl) , Lithiumbromid (LiBr), Kaliumchlorid (KCl), Natriumchlorid (NaCl) und Cäsiumchlorid (CsCl), wobei Lithiumchlorid bevorzugt ist.

Ganz besonders bevorzugt ist die Kombination von AlCl₃ und LiCl, besonders bevorzugt in den oben genannten und in Vorzugsbereichen ausgeführten Verhältnissen.

In den genannten Vorzugsbereichen wird vorteilhaft bewerkstelligt, dass die über die Verbindungen der Formel (II) eingebrachte Lewis-Acidität hoch ist und der Schmelzpunkt der Salzschmelze so niedrig ist, dass die Isomerisierung bei Temperaturen durchgeführt werden kann, die die Verbindungen der Formel (I) zumindest weitestgehend unzersetzt lässt.

In Tabelle 1 sind Beispiele für niedrig schmelzende Salzschmelzen dargestellt, die sich als Katalysatorsysteme für die Isomerisierung besonders eignen.

**Tabelle 1: Schmelzpunkte binärer und ternärer Salzschmelzen**

| Molare Zusammensetzung | Schmelzpunkt [°C] |
|---|---|
| AlCl₃/LiCl (2:1) | 170* |
| AlCl₃/CsCl (2:1) | 150* |
| AlCl₃/KCl(2:1) | 160** |
| AlCl₃/KCl/NaCl (66 mol % / 14 mol % / 20 mol %) | 130** |
| GaCl₃-LiCl (2:1) | 120*** |
| AlBr₃-LiBr (2:1) | 150*** |

| | |
|---|---|
| Verbindungen der Formeln (II) * Morozov, Zh. Neorg. Khim. 1957, 2, 1906. ** Robelin, Chartrand, Pelton, J. Chem. Thermodynamics 2004, 36, 683-699 *** eigene Messungen | |

Es wurde weiterhin festgestellt, dass die Gegenwart von Protonen für die Isomerisierung förderlich ist. Die dadurch verursachte Bronsted-Acidität ergänzt die Lewis-Acidität der Verbindungen der Formel (II).

Der Gehalt an Protonen beträgt beispielsweise 1 mol-ppm bis 50.000 mol-ppm bezogen auf Verbindungen der Formel (II) bevorzugt 10 bis 1.000 mol-ppm. Höhere Mengen stören die Isomerisierung vermutlich durch abnehmende Lewis-Acidität.

Unter Protonen sind hierbei solche Protonen zu verstehen, die aus Verbindungen stammen, die eben diese Protonen mit einem pKs-Wert von 5 oder weniger, vorzugsweise 2 oder weniger und besonders bevorzugt 1 oder weniger bei 25°C bezogen auf ein wässriges System oder wässriges Bezugssystem, freisetzen können.

Typischerweise ist der gesonderte Zusatz von entsprechenden Verbindungen, die diese Protonen liefern, nicht erforderlich, da die eingesetzten Verbindungen der Formeln (II) und (III) typischerweise nie absolut wasserfrei sind. Wasser reagiert typischerweise mit Verbindungen der Formel (II) unter Bildung entsprechend saurer Verbindungen. So enthält beispielsweise Aluminiumchlorid durch partielle Hydrolyse mit geringsten Mengen Wasser typischerweise immer Spuren von Chlorwasserstoff, der aufgrund seines niedrigen pKs Wertes von -6 entsprechende Protonen bereitstellt.

Vorzugsweise beträgt das molare Verhältnis von Verbindungen der Formel (I) zu Verbindungen der Formel (II) 1:1000 bis 100:1, vorzugsweise 1:1 1 bis 10:1, besonders bevorzugt 20:1 bis 3:1, und ganz besonders bevorzugt 2:1 bis 5:1.

Die Reaktionstemperatur bei der Isomerisierung beträgt beispielsweise zwischen 100 °C und 220 °C gewählt werden, wobei eine niedrigere Temperatur zu einer unvorteilhaften Verlangsamung der Reaktion führt und eine zu hohe Temperatur Sublimation von einigen Verbindungen der Formel (II) wie beispielsweise von Aluminiumchlorid sowie je nach Art der Verbindung der Formel (I) Zersetzung derselben fördert.

Vorzugsweise beträgt die Reaktionstemperatur bei der Isomerisierung 120 bis 200°C, besonders bevorzugt 140 bis 180 °C durchgeführt. Bei Dihalogenbenzolen ist beispielsweise eine Temperatur von 160 bis 180°C und bei Methylhalogenbenzolen eine Temperatur von 140 bis 160°C besonders vorteilhaft.

Die der Isomerisierung liegen aufgrund der unterschiedlichen Natur der eingesetzten Verbindungen der Formeln (I) bis (III) typischerweise und vorzugsweise zwei flüssige Phasen vor, wobei die erste Phase von den Verbindungen der Formel (I) gebildet wird und die zweite Phase von den Verbindungen der Formeln (II) und (III).

Vorzugsweise werden während der Isomerisierung die beiden Phasen durch Einbringung von Mischenergie miteinander vermischt, um die Phasengrenzflächen zu erhöhen. Je intensiver dies erfolgt desto schneller verläuft typischerweise die Reaktion.

Die Reaktionsdauer der Isomerisierung beträgt üblicherweise zwischen 10 min und 168 h, vorzugsweise zwischen 30 min und 24 h.

Nach erfolgter Reaktion kann das erhaltene Produktgemisch das aus den Verbindungen der Formel (I) erhalten wurde von den Verbindungen der Formeln (II) und (III) abgetrennt werden, vorzugsweise solange sie noch eine Schmelze bilden und nicht erstarrt sind.

Überraschenderweise wurde gefunden, dass die Löslichkeit der Verbindungen der Formeln (II) und (III) in den Verbindungen der Formel (I) oder ihren Isomerisierungsprodukten so gering ist, dass weniger als 10% der Verbindungen der Formeln (II) in die Substratphase ausgetragen werden, üblicherweise sogar weniger als 1%. Austrag von Verbindungen der Formel (III) wird typischerweise nicht beobachtet.

Die Verbindungen der Formel (II) und (III) können daher für eine erneute Isomerisierung verwendet werden.

Es wurde gefunden, dass die Zuführung von Protonen zu den Verbindungen der Formel (II) und (III) vor oder während ihrem erneuten Einsatz vorteilhaft ist.

Unter Protonen sind hierbei wie oben solche Protonen zu verstehen, die aus Verbindungen stammen, die eben diese Protonen mit einem pKs-Wert von 5 oder weniger, vorzugsweise 2 oder weniger und besonders bevorzugt 1 oder weniger bei 25°C bezogen auf ein wässriges System oder wässriges Bezugssystem, freisetzen können.

Vorzugsweise werden daher die Verbindungen der Formel (II) und (III) vor oder während ihrem erneuten bzw. wiederholten Einsatz mit Chlorwasserstoff oder Bromwasserstoffgas in Kontakt gebracht, wobei der Partialdruck dieser Gase beispielsweise 50 hPa bis 1 MPa, vorzugsweise 500 hPa bis 2500 hPa betragen kann.

Das Verfahren kann grundsätzlich jedoch auch ohne Zuführung von Protonen zu den Verbindungen der Formel (II) und (III) vor oder während ihrem erneuten Einsatz erfolgen.

Vorteil der Erfindung ist, dass eine aufwändige Extraktion der Produktphase oder Aufarbeitung mit Wasser entfällt und der Isomerisierungskatalysator mehrfach ohne Aktivitätsverlust verwendet werden kann.

### Beispiele:

Für die Analyse der Produktphase der folgenden Beispiele wird eine Probe direkt aus dem Reaktionsgemisch entnommen, in Dichlormethan gelöst und mit Wasser extrahiert. Zur Bestimmung des Isomerenverhältnisses wird die Probe mittels Gaschromatographie (GC) analysiert. Alle Angaben beziehen sich auf GC-Flächenprozent. Die nachfolgenden Angaben mit mol-% beziehen sich auf die Salzkomponenten in Bezug auf das jeweils eingesetzte Substrat, das isomerisiert wurde.

### Beispiel 1a: Isomerisierung von para-Dichlorbenzol mit AlCl₃/LiCl in einem Glas-Autoklaven

In einem 0.51-Glas-Autoklaven mit mechanischem Rührer wurden 20 mol-% Aluminiumchlorid und 10 mol-% Lithiumchlorid bei 170°C zum Schmelzen gebracht und mit 250 g para-Dichlorbenzol versetzt. Die Reaktionslösung wurde bei 170 °C für 4 h gerührt. Nach Beendigung der Reaktionszeit wurde eine Zusammensetzung von 53 % meta-Dichlorbenzol, 44 % para-Dichlorbenzol und 3 % ortho-Dichlorbenzol im Produktgemisch festgestellt. Die Produktphase wurde mittels ICP-AES auf ausgetragenes Aluminium und Lithium hin untersucht. Der Austrag des Aluminiums betrug typischerweise weniger als 1% im Bezug auf das eingesetzte Aluminiumchlorid. Ein Austrag von Lithium konnte nicht beobachtet werden.

### Beispiel 1b: Rezyklierung der Salzschmelze

Für die Rezyklierung der Schmelze wurde die Produktphase abgetrennt. Das ausgetragene Aluminiumchlorid wurde ersetzt und 250 g neues para-Dichlorbenzol hinzugefügt. Anschließend wurde eine Reaktionstemperatur von 170 °C eingestellt. Vor Beginn der Rührung wurde ein Chlorwasserstoff-Druck von etwa 1,0 bar angelegt. Die Aktivität der Salzschmelze blieb über mindestens 5 Rezyklierungen konstant.

**Tabelle 2: Produktzusammensetzung nach der Rezyklierung**

| | **meta-Dichlorbenzol [%]** | **para-Dichlorbenzol [%]** | **ortho-Dichlorbenzol [%]** |
|---|---|---|---|
| 1. Durchlauf | 53 | 44 | 3 |
| 1. Rezyklierung | 50 | 47 | 3 |
| 2. Rezyklierung | 52 | 44 | 4 |
| 3. Rezyklierung | 52 | 44 | 4 |
| 4. Rezyklierung | 54 | 41 | 5 |
| 5. Rezyklierung | 57 | 38 | 5 |

### Beispiel 1c: Druckvariation bei der Rezyklierung der Salzschmelze

Zur Druckvariation bei der Rezyklierung im 0,5-1-Glasreaktor, wurde bei der 1. Rezyklierung ein Druck von 0,05 bis 0,25 MPa verwendet. Hierfür wurde nach der Isomerisierung mir der frischen Salzschmelze die Produktphase abgetrennt, das ausgetragene Aluminiumchlorid ersetzt und 250 g neues para-Dichlorbenzol hinzugefügt. Anschließend wurde eine Reaktionstemperatur von 170 °C eingestellt. Vor Beginn der Rührung wurde ein Chlorwasserstoff-Druck von 0,05 bis 0,25 MPa angelegt. Die nach 4 h Reaktionszeit erhaltenen Ergebnisse sind in Tabelle 3 dargestellt.

**Tabelle 3: Produktzusammensetzung nach der 1. Rezyklierung bei unterschiedlichem HCl-Druck**

| **HCl Druck [hPa]** | **meta-Dichlorbenzol [%]** | **para-Dichlorbenzol [%]** | **ortho-Dichlorbenzol [%]** |
|---|---|---|---|
| 0 | 35 | 63 | 2 |
| 500 | 41 | 57 | 2 |
| 1000 | 50 | 46 | 4 |
| 1500 | 54 | 43 | 3 |
| 2000 | 58 | 38 | 4 |
| 2500 | 60 | 34 | 6 |

### Beispiel 1d: Produktabtrennung

Nach Beendigung der Reaktion wurde die organische Phase von der Schmelze abgetrennt. Das Produktgemisch bestand im Wesentlichen aus den drei Isomeren des Dichlorbenzols, aus kleinen Mengen ausgetragenem Aluminiumchlorid und kleinen Mengen von Nebenprodukten. Das Dichlorbenzol ließ sich quantitativ durch Flash-Destillation abtrennen. Im Sumpf verblieben dabei sowohl das Aluminiumchlorid als auch die Nebenprodukte. Der Anteil der Nebenprodukte lag bei unter 1 Gew.-%.

### Beispiel 2: Isomerisierung von para-Dichlorbenzol mit AlCl₃/LiCl unter Zugabe von Lewis-Säuren

In einem 250-ml-Glas-Glaskolben mit mechanischem Rührer wurden 20 mol-% Aluminiumchlorid und 10 mol-% Lithiumchlorid bei 180 °C **aufgeschmolzen** und mit a) 1,5 mol-% FeCl₃, b) 1,5 mol-% NiCl₂, c) 1 mol-% CuCl₂ versetzt. Den dadurch entstandenen Salzschmelzen wurden jeweils 150 g para-Dichlorbenzol zugesetzt, und das Reaktionsgemisch bei 180 °C isomerisiert. In allen Experimenten wurde nach 4 h ein Anteil von ungefähr 20 % meta-Dichlorbenzol erhalten.

### Beispiel 3: Isomerisierung von para-Dichlorbenzol mit AlCl₃/NaCl/KCl bei 150°C

In einem Hastelloy-Reaktionsgefäß wurden 20 mol-% Aluminiumchlorid, 6,25 mol-% Natriumchlorid und 4,37 mol-% Kaliumchlorid bei 150 °C aufgeschmolzen und mit 75 g para-Dichlorbenzol versetzt. Die Reaktionslösung wurde bei 150 °C für 3 h gerührt. Nach Beendigung der Reaktion konnte ein Anteil von 26 % meta-Dichlorbenzol und 74 % para-Dichlorbenzol festgestellt werden.

### Beispiel 4: Temperaturabhängigkeit der Isomerisierung von para-Dichlorbenzol mit AlCl₃/NaCl/LiCl

In einem Hastelloy-Reaktionsgefäß wurden 20 mol-% Aluminiumchlorid, 6,25 mol% Natriumchlorid und 4,37 mol-% Lithiumchlorid bei a) 110 °C, b) 130 °C und c) 150 °C aufgeschmolzen und mit 75 g para-Dichlorbenzol versetzt. Die Reaktionslösung wurde bei der jeweiligen Reaktionstemperatur für 16,5 h gerührt. Nach Beendigung der Reaktion konnte im Experiment a) ein Anteil von 13 % meta-Dichlorbenzol und 87 % para-Dichlorbenzol, im Experiment b) ein Anteil von 44 % meta-Dichlorbenzol und 55 % para-Dichlorbenzol, 1 % ortho-Dichlorbenzol und im Experiment c) jeweils ein Anteil von 58 % meta-Dichlorbenzol und 37 % para-Dichlorbenzol und 5 % ortho-Dichlorbenzol ermittelt werden.

### Beispiel 5: Isomerisierung von para-Dichlorbenzol mit GaCl₃/LiCl bei 170°C

50-ml-Glaskolben mit mechanischem Rührer wurden 20 mol-% Galliumchlorid mit 10 mol-% Lithiumchlorid bei 170 °C zum Schmelzen gebracht. Nach der Zugabe von 20,87 g para-Dichlorbenzol wurde die Reaktionslösung für 25 h gerührt. Nach Beendigung der Reaktion enthält das Reaktionsgemisch einen Anteil von 3 % meta-Dichlorbenzol und 97 % para-Dichlorbenzol.

### Beispiel 6a: Isomerisierung von ortho-Dichlorbenzol mit AlCl₃/LiCl

In einem 0.51-Glas-Autoklaven mit mechanischem Rührer wurden 20 mol-% Aluminiumchlorid und 10 mol-% Lithiumchlorid bei 170°C zum Schmelzen gebracht und mit 250 g ortho-Dichlorbenzol versetzt. Die Reaktionslösung wurde bei 170 °C für 3,5 h gerührt. Nach Beendigung der Reaktionszeit wurde eine Zusammensetzung von 31 % meta-Dichlorbenzol, 9 % para-Dichlorbenzol und 60 % ortho-Dichlorbenzol im Produktgemisch festgestellt.

### Beispiel 6b: Rezyklierung der Salzschmelze bei der Isomerisierung von ortho-Dichlorbenzol

Für die Rezyklierung der Schmelze wurde die Produktphase abgetrennt. Das ausgetragene Aluminiumchlorid wurde ersetzt und 250 g neues ortho-Dichlorbenzol hinzugefügt. Anschließend wurde eine Reaktionstemperatur von 170 °C eingestellt. Vor Beginn der Rührung wurde ein Chlorwasserstoff-Druck von etwa 1,0 bar angelegt. Nach Beendigung der Reaktionszeit wurde eine Zusammensetzung von 26 % meta-Dichlorbenzol, 9 % para-Dichlorbenzol und 65 % ortho-Dichlorbenzol im Produktgemisch festgestellt.

### Beispiel 7: Isomerisierung von 1,2,3-Trichlorbenzol mit AlCl₃/LiCl und AlBr₃/LiBr

In einem 50-ml-Glaskolben mit mechanischem Rührer wurden a) 20 mol-% Aluminiumchlorid mit 10 mol-% Lithiumchlorid und b) 20 mol-% Aluminiumbromid mit 10 mol-% Lithiumbromid bei 170 °C zum Schmelzen gebracht. Nach Zusatz von 36,29 g 1,2,3-Trichlorbenzol wurde die Reaktionslösung für 22 h bei 170 °C gerührt. Nach Beendigung der Reaktion ergab sich eine Zusammensetzung der Isomere im Experiment a) von 5 % 1,2,4-Trichlorbenzol und 95 % 1,2,3-Trichlorbenzol. Im Experiment b) lag der Anteil von 1,2,4-Trichlorbenzol bei 27 % und der Anteil von 1,2,3-Trichlorbenzol bei 73 %. Allerdings entsteht beim Experiment b) auch eine gewisse Anzahl an Nebenprodukten. Das Verhältnis von Trichlorbenzol zu Nebenprodukten lag bei 86 % zu 14 %. Die Nebenprodukte bestanden zum einen aus Dichlorbenzolen, zu einem gewissen Anteil aber auch aus höher chlorierten Isomeren.

### Beispiel 8: Isomerisierung von para-Chlortoluol mit AlCl₃/LiCl

In einem 50-ml-Glaskolben mit mechanischem Rührer wurden 20 mol-% Aluminiumchlorid mit 10 mol-% Lithiumchlorid bei 170 °C **aufgeschmolzen** und mit 25,14 g para-Chlortoluol versetzt. Nach einer Reaktionszeit von 20 Minuten ergab sich ein Isomerenverhältnis von 49% meta-Chlortoluol, 45 % para-Chlortoluol und 6 % ortho-Chlortoluol. Neben der Isomerisierung fand auch eine Transalkylierung statt. So ergaben sich als Nebenprodukte neben Chlorbenzol auch höher alkylierte Verbindungen, wie Chlorxylole. Das Verhältnis von Chlortoluolen zu Nebenprodukten lag bei 86 % zu 14 %.

### Beispiel 9: Isomerisierung von para-Chlorbrombenzol mit AlCl₃/LiCl und AlBr₃/LiBr

In einem 50-ml-Glaskolben mit mechanischem Rührer wurden a) 20 mol-% Aluminiumchlorid mit 10 mol-% Lithiumchlorid und b) 20 mol-% Aluminiumbromid mit 10 mol-% Lithiumbromid bei 170 °C zum Schmelzen gebracht. Nach der Zugabe von 38,29 g para-Bromchlorbenzol wurde das Reaktionsgemisch für 1 h bei 170 °C gerührt. Im Experiment a) ergab sich eine Isomerenverteilung von 59 % meta-Bromchlorbenzol 30 % para-Bromchlorbenzol und 11 % ortho-Bromchlorbenzol. Neben der Isomerisierung fand auch eine Disproportionierung statt. Entstehende Nebenprodukte waren zum einen Chlorbenzol als auch Dibromchlorbenzole. Das Verhältnis von Chlorbrombenzolen zu Nebenprodukten betrug 67 % zu 33 %. Im Experiment b) ergab sich nach 1 h Reaktion eine Isomerenverteilung von 57 % meta-Bromchlorbenzol 32 % para-Bromchlorbenzol und 11 % ortho-Bromchlorbenzol. Auch hier entstanden dieselben Nebenprodukte wie im Experiment a), ebenfalls mit einem Verhältnis Chlorbrombenzolen zu Nebenprodukten von 67 % zu 33 %.

### Beispiel 10: Isomerisierung von para-Dichlorbenzol mit AlBr₃/LiBr

In einem 50-ml-Glaskolben mit mechanischem Rührer wurden 20 mol-% Aluminiumbromid mit 10 mol-% Lithiumbromid bei 170 °C zum Schmelzen gebracht. Zur Schmelze wurden anschließend 29,40 g para-Dichlorbenzol hinzugefügt. Nach einer Reaktionszeit von 23 h ergab sich eine Zusammensetzung von 32 % meta-Dichlorbenzol und 68 % para-Dichlorbenzol. Die Anwesenheit von Chlorbenzol, Trichlorbenzolen, Bromchlorbenzolen oder Dibrombenzolen konnte nicht nachgewiesen werden. Im Vergleich mit der Aluminiumchlorid-Lithiumchlorid-Schmelze, zeigt bei vergleichbaren Experimenten die Aluminiumbromid-Schmelze eine höhere Aktivität.

### Beispiel 11: Isomerisierung von para-Dibrombenzol mit AlCl₃/LiCl und AlBr₃/LiBr

In einem 50-ml-Glaskolben mit mechanischem Rührer wurden a) 20 mol-% Aluminiumchlorid mit 10 mol-% Lithiumchlorid und b) 20 mol-% Aluminiumbromid mit 10 mol-% Lithiumbromid bei 170 °C zum Schmelzen gebracht. Anschließend wurden 47,18 g para-Dibrombenzol zugesetzt. Die Reaktionslösung wurde bei 170 °C für 5 h gerührt. Im Experiment a) ergab sich nach 1 h Reaktionszeit ein Verhältnis der Isomere von 59 % meta-Dibrombenzol, 31 % para-Dibrombenzol und 10 % ortho-Dibrombenzol im Reaktionsgemisch. Allerdings entstanden auch in größeren Mengen Brombenzol und Tribrombenzole sowie in Spuren Bromchlorbenzole, da neben der Isomerisierung auch eine Disproportionierung stattfand. Das Verhältnis der Dibrombenzole zu Nebenprodukten lag bei 62 % zu 38 %. Im Experiment b) ergab sich nach 1 h Reaktionszeit eine Zusammensetzung von 59 % meta-Dibrombenzol 31 % para-Dibrombenzol und 10 % ortho-Dibrombenzol. Auch hier entstanden Brombenzol und Tribrombenzole als Nebenprodukte. Das Verhältnis der Dibrombenzole zu Nebenprodukten lag bei 60 % zu 40 %.

## Patentansprüche

1. Verfahren zur Isomerisierung von Verbindungen der Formel (I) oder Mischungen von Verbindungen der Formel (I)
Ar-Rₙ (I)
worin
Ar für einen n-valenten aromatischen Rest steht,
n für eine natürliche Zahl von 2 oder mehr, vorzugsweise 2 oder 3 und besonders bevorzugt 2 steht und
R jeweils unabhängig für Halogen, Alkyl, Fluoralkyl, Aryl, Alkyl-Aryl oder Amino steht,
**dadurch gekennzeichnet, dass** die Isomerisierung in Gegenwart einer Salzschmelze erfolgt, die enthält:
• zumindest eine Verbindung der Formel (II): [M¹][X¹]ₘ₁
in der
M¹ für Aluminium, Gallium, Indium, Kupfer, Eisen, Cobalt, Nickel oder Kupfer,
X¹ für ein Halogen, vorzugsweise Chlor oder Brom, besonders bevorzugt Chlor steht und
m1 für Aluminium, Gallium, Indium, Eisen für 3, für Cobalt, Nickel und Kupfer für 2 steht
• zumindest eine Verbindung der Formel (III):
[M²][X²]ₘ₂
in der
M² für ein Erdalkali- oder Alkalimetall, vorzugsweise ein Alkalimetall und besonders bevorzugt für Lithium, Natrium oder Kalium steht, wobei Lithium noch weiter bevorzugt ist,
X² für ein Halogen, vorzugsweise Chlor oder Brom, besonders bevorzugt Chlor steht und
m2 für Erdalkalimetalle für 2, für Alkalimetalle für 1 steht.

2. Verfahren nach Anspruch 1, **dadurch gekennzeichnet, dass** in Formel (I) Ar steht für:
carbocyclische aromatische Reste mit 6 bis 24 Gerüstkohlenstoffatomen oder für heteroaromatische Reste mit 5 bis 24 Gerüstatomen, in denen keines, ein, zwei oder drei Gerüstatome pro Cyclus, im gesamten Molekül mindestens jedoch ein Gerüstatom Heteroatome sind, die ausgewählt sind aus der Gruppe Stickstoff, Schwefel oder Sauerstoff.

3. Verfahren nach Anspruch 1 oder 2, **dadurch gekennzeichnet, dass** in Formel (I) Ar steht für:
aromatische Reste, die sich vom Benzol, Naphtalin, Phenanthren, Anthracen, Biphenyl, Binaphthyl, Fluoren, Pyridin, Oxazol, Thiophen, Benzofuran, Benzothiophen, Dibenzofuran, Dibenzothiophen, Furan, Indol, Pyridazin, Pyrazin, Pyrimidin, Triazol oder
Chinolin ableiten, ganz besonders bevorzugt für aromatische Reste, die sich vom Benzol oder Naphthalin ableiten, wobei ein aromatischer Rest der sich von Benzol ableitet, noch weiter bevorzugt ist.

4. Verfahren nach einem der Ansprüche 1 bis 3, **dadurch gekennzeichnet, dass** als Verbindungen der Formel (I) solche eingesetzt werden, in denen n = 2 ist und ein oder zwei der Reste Halogene wie vorzugsweise Brom oder Chlor, besonders bevorzugt Chlor sind oder Verbindungen der Formel (I), in denen n = 3 ist und zwei oder drei der Reste Halogene wie vorzugsweise Brom oder Chlor, besonders bevorzugt Chlor sind.

5. Verfahren nach einem der Ansprüche 1 bis 4, **dadurch gekennzeichnet, dass** Isomere von Dichlorbenzol, vorzugsweise p-Dichlorbenzol und o-Dichlorbenzol, Trichlorbenzol, Chlortoluol, Dibrombenzol oder Bromchlorbenzol eingesetzt werden.

6. Verfahren nach einem der Ansprüche 1 bis 5, **dadurch gekennzeichnet, dass** das molare Verhältnis von Verbindungen der Formel (II) zu Verbindungen der Formel (III) 1:1 bis 10:1, vorzugsweise 1:1 bis 3:1 und besonders bevorzugt 1,8:1 bis 2,2:1 beträgt, wobei 2:1 noch weiter bevorzugt ist.

7. Verfahren nach einem der Ansprüche 1 bis 6, **dadurch gekennzeichnet, dass** die Verbindungen der Formel (II) ausgewählt sind aus der Gruppe Aluminiumchlorid, Aluminiumbromid und Galliumchlorid, wobei Aluminiumchlorid bevorzugt ist.

8. Verfahren nach einem der Ansprüche 1 bis 7, **dadurch gekennzeichnet, dass** die Verbindungen der Formel (III) ausgewählt sind aus der Gruppe Lithiumchlorid, Lithiumbromid, Kaliumchlorid, Natriumchlorid und Cäsiumchlorid, wobei Lithiumchlorid bevorzugt ist.

9. Verfahren nach einem der Ansprüche 1 bis 8, **dadurch gekennzeichnet, dass** es in Gegenwart von Protonen durchgeführt wird, die aus Verbindungen stammen, die eben diese Protonen mit einem pKs-Wert von 5 oder weniger, vorzugsweise 2 oder weniger und besonders bevorzugt 1 oder weniger bei 25°C bezogen auf ein wässriges System oder wässriges Bezugssystem, freisetzen können.

10. Verfahren nach einem der Ansprüche 1 bis 9, **dadurch gekennzeichnet, dass** das molare Verhältnis von Verbindungen der Formel (I) zu Verbindungen der Formel (II) 1:1000 bis 100:1, vorzugsweise 1:1 bis 10:1, besonders bevorzugt 20:1 bis 3:1, und ganz besonders bevorzugt 2:1 bis 5:1 beträgt.

11. Verfahren nach einem der Ansprüche 1 bis 10, **dadurch gekennzeichnet, dass** die Reaktionstemperatur bei der Isomerisierung zwischen 100 °C und 220 °C, vorzugsweise 120 bis 200°C und besonders bevorzugt 140 bis 180 °C beträgt.

12. Verfahren nach einem der Ansprüche 1 bis 11, **dadurch gekennzeichnet, dass** die bei der Duchführung des Verfahrens entstehenden zwei flüssigen Phasen durch Einbringung von Mischenergie miteinander vermischt werden.

13. Verfahren nach einem der Ansprüche 1 bis 12, **dadurch gekennzeichnet, dass** die Verbindungen der Formel (II) und (III) erneut in das Verfahren eingesetzt werden.

14. Verfahren nach Anspruch 13, **dadurch gekennzeichnet, dass** zu den Verbindungen der Formel (II) und (III) vor oder während ihrem erneuten Einsatz Protonen zugeführt werden, die aus Verbindungen stammen, die eben diese Protonen mit einem pKs-Wert von 5 oder weniger, vorzugsweise 2 oder weniger und besonders bevorzugt 1 oder weniger bei 25°C bezogen auf ein wässriges System oder wässriges Bezugssystem, freisetzen können.

15. Verfahren nach Anspruch 13 oder 14, **dadurch gekennzeichnet, dass** die Verbindungen der Formel (II) und (III) vor oder während ihrem erneuten Einsatz mit Chlorwasserstoff oder Bromwasserstoffgas in Kontakt gebracht werden, wobei der Partialdruck dieser Gase vorzugsweise 50 hPa bis 1 MPa, besonders bevorzugt 500 hPa bis 2500 hPa beträgt.
